# EUROPEAN PATENT SPECIFICATION

(11) **EP 2 926 727 B1**
(45) Date of publication and mention of the grant of the patent: **27.12.2023**
(21) Application number: 13867439.5
(22) Date of filing: 01.10.2013
(51) Int. Cl.: A61B 5/024, A61B 5/00, A61B 5/11, G16H 20/30

(54) **ELECTRONIC DEVICE AND PROGRAM**
ELEKTRONISCHE VORRICHTUNG UND PROGRAMM
DISPOSITIF ÉLECTRONIQUE ET PROGRAMME

(30) Priority: 28.12.2012 JP 2012288951
(43) Date of publication of application: 07.10.2015
(73) Proprietor: Seiko Instruments Inc., Chiba-shi, Chiba 261-8507 (JP); ASICS Corporation, Kobe-shi, Hyogo 650-8555 (JP)
(72) Inventor: TAKAKURA, Akira, Chiba-shi Chiba 261-8507 (JP); HASEGAWA, Takanori, Chiba-shi Chiba 261-8507 (JP); KOYAMA, Kazuhiro, Chiba-shi Chiba 261-8507 (JP); TSUBATA, Keisuke, Chiba-shi Chiba 261-8507 (JP); TAGAWA, Takehiro, Kobe-shi Hyogo 650-8555 (JP); SHINAYAMA, Ryota, Kobe-shi Hyogo 650-8555 (JP)
(74) Representative: Lewis Silkin LLP
(86) International application number: PCT/JP2013/076708
(87) International publication number: WO 2014/103458

(56) References cited:
- EP-A1- 0 748 185
- JP-A- H07 213 499
- JP-A- 2008 229 200
- US-A- 5 301 154
- US-A1- 2007 060 446
- TAKAHIKO NOHARA ET AL: "Studies of the Relationship between OBLA Speed and Heart Rate during Distance Running distance running, OBLA speed, heart rate", BULLETIN OF SHIMANE MEDICAL UNIVERSITY, vol. 26, 1 September 2003 (2003-09-01), pages 1-9, XP055258056,
- TAKAHIKO NOHARA ET AL.: 'Studies of the relationship between OBLA speed and heart rate during distance running' BULLETIN OF SHIMANE MEDICAL UNIVERSITY vol. 26, 01 September 2003, pages 1 - 9, XP055258056
- SETSUO KOMAI ET AL.: 'Relationship of Running Preformance and Aerobic Parameters of Distance Runners' RESEARCH REPORTS OF KOCHI UNIVERSITY. NATURAL SCIENCE vol. 40, 1991, pages 169 - 179, XP008180172
- KEIJI YAMAJI: 'Significance and Assessment of the Velocity at Vo2 (v Vo2max' JOURNAL OF EXERCISE AND SPORTS PHYSIOLOGY vol. 5, no. ISS. 1, 1998, pages 89 - 99, XP008180163

## Description

### TECHNICAL FIELD

The present invention relates to an electronic apparatus and a program. The present application claims priority based on Japanese Patent Application No. 2012-288951, filed on December 28, 2012.

### BACKGROUND ART

In recent years, an electronic apparatus which displays a heart rate is known. In addition, an electronic apparatus which displays an elapsed time from a starting of measurement to an ending of the measurement is known. For example, Patent Document 1 discloses a technique in which a value proportional to a spectrum based on the variation in a heart rate or a heart rate is supplied, a value proportional to a heart rate variation value is recorded as a function of time, an exercise level in a training period is calculated and displayed on the basis of the heart rate variation value. Another known electrical apparatus is disclosed in US5301154.

### RELATED ART DOCUMENT

### PATENT DOCUMENT

[Patent Document 1] Japanese Patent No. 3831410

### SUMMARY OF INVENTION

### PROBLEMS TO BE SOLVED BY THE INVENTION

In order to run a full marathon, it is important to maintain a running pace, with an anaerobic threshold (AT) heart rate (hereinafter, referred to as an AT heart rate) as a reference. In order to learn about an AT heart rate of a person, it is necessary to measure a boundary between an aerobic exercise and an anaerobic exercise, and thus dedicated equipment is generally necessary. For this reason, an AT heart rate cannot be easily measured. As mentioned above, a reference heart rate which is used as a reference of a heart rate, such as an AT heart rate, cannot be easily measured, and a user's burden is considerable.

Therefore, some aspects of the present invention are to provide an electronic apparatus and a program capable of reducing a user's burden when a reference heart rate is determined.

### MEANS FOR SOLVING THE PROBLEM

(1) According to some aspects of the present invention, there is provided an electronic apparatus in accordance with claim 1.
(12) According to still another aspect of the present invention, there is provided a program stored on a non-transitory computer-readable recording medium in accordance with claim 9.

### ADVANTAGE OF THE INVENTION

According to some aspects of the present invention, it is possible to reduce a user's burden when a reference heart rate is determined.

### BRIEF DESCRIPTION OF THE DRAWINGS

FIG. 1 is a schematic configuration diagram illustrating an apparatus configuration of a running watch system in an embodiment of the present invention.
FIG. 2 is a schematic block diagram illustrating a configuration of the running watch system.
FIG. 3 is a schematic block diagram illustrating a functional configuration of a processing section in the present embodiment.
FIG. 4 is a diagram for explaining a concept of a process performed by a reference running speed determination unit.
FIG. 5 is a diagram for explaining a concept of a process performed by a determination unit.
FIG. 6 illustrates an example of a display screen in a case where the present heart rate is higher than an AT zone.
FIG. 7 illustrates an example of a display screen in a case where the present heart rate is within the AT zone.
FIG. 8 illustrates an example of a display screen in a case where the present heart rate is lower than the AT zone.
FIG. 9 is a flowchart illustrating an example of a flow of a process in the running watch system according to the present embodiment.
FIG. 10 illustrates a first application example of a display screen of a display section.
FIG. 11 illustrates a second application example of a display screen of the display section.
FIG. 12 illustrates a third application example of a display screen of the display section.
FIG. 13 is a diagram for explaining a concept of a process performed by the reference running speed determination unit in Modification Example 1.
FIG. 14 is a diagram for explaining a concept of a process performed by the reference running speed determination unit in Modification Example 2.

### DESCRIPTION OF THE EMBODIMENTS

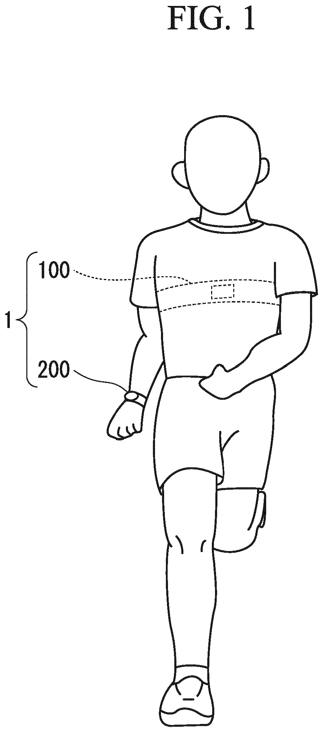

Hereinafter, an embodiment of the present invention will be described in detail with reference to the drawings. FIG. 1 is a schematic configuration diagram illustrating an apparatus configuration of a running watch system 1 in an embodiment of the present invention. In FIG. 1, the running watch system 1 includes a chest strap 100 and a running watch 200.

The running watch system 1 provides information such as a heart rate or a lap time to, for example, a user who is playing sports such as running. Hereinafter, in the present embodiment, a heart rate per minute will be described as a heart rate as an example.

The chest strap 100 is mounted on, for example, a user's chest, detects heartbeats of the user, and wirelessly transmits a heartbeat signal which is synchronized with the detected heartbeats to the running watch 200. The running watch 200 displays information such as a heart rate or a lap time and thus provides such information to the user. Specifically, for example, the running watch 200 receives a heartbeat signal from the chest strap 100, and displays information indicating a heart rate on the basis of the received heartbeat signal. In addition, the running watch 200 displays, for example, the time, a lap time, or a split time on a display screen. In the present embodiment, the running watch 200 corresponds to an example of an electronic apparatus in the present invention.

In addition, in the present embodiment, an electronic apparatus will be described by exemplifying the running watch 200 but is not limited thereto, and an electronic apparatus may be a treadmill.

Further, in the present invention, a form in which a measurement device transmits a biological signal to an electronic apparatus is not limited to wireless communication, but wired communication may be employed. Still further, a measurement device may be built into an electronic apparatus.

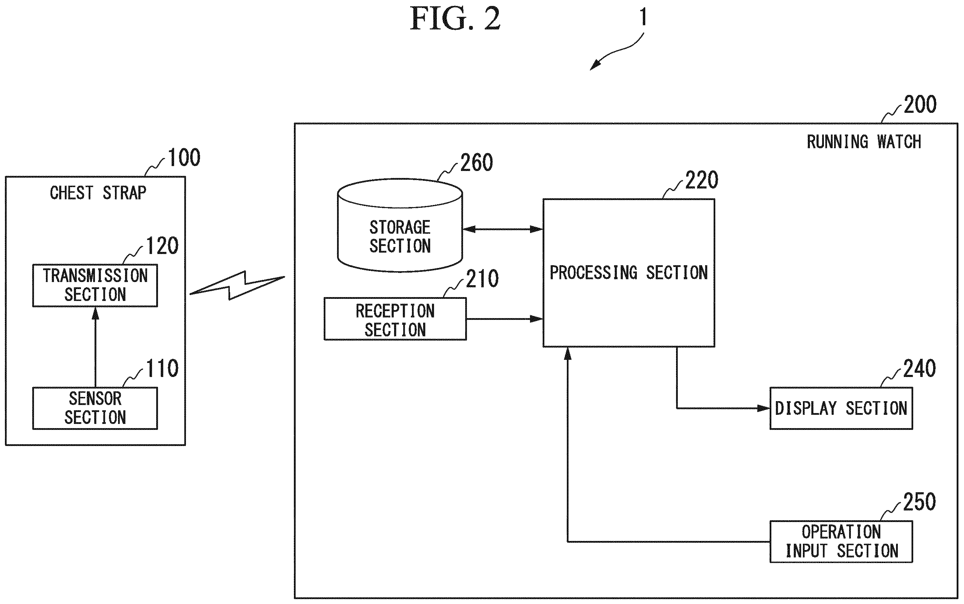

FIG. 2 is a schematic block diagram illustrating a configuration of the running watch system 1. In FIG. 2, the running watch system 1 includes the chest strap 100 and the running watch 200. The chest strap 100 includes a sensor section 110 and a transmission section 120. The running watch 200 includes a reception section 210, a processing section 220, a display section 240, an operation input section 250, and a storage section 260.

The sensor section 110 has a heartbeat sensor, detects heartbeats of the user, and outputs a heartbeat signal which is synchronized with the detected heartbeats to the transmission section 120. The heartbeat signal is a signal indicating 1 in a case of exceeding, for example, a predefined threshold value, and indicating 0 otherwise. In the present embodiment, as an example, heartbeats obtained when the user runs 5 km is detected, and a heartbeat signal synchronized with the detected heartbeats is output to the transmission section 120.

The transmission section 120 transmits the heartbeat signal which has been output from the sensor section 110, to the running watch 200 by using communication means such as wireless means.

The reception section 210 receives the heartbeat signal which is transmitted from the transmission section 120 of the chest strap 100 in a wireless manner, and outputs the received heartbeat signal to the processing section 220.

The processing section 220 calculates a heart rate on the basis of the heartbeat signal received by the reception section 210, and displays the calculated heart rate on a display screen. In addition, the processing section 220 measures a time period from the time when the operation input section 250 receives an input operation for starting a measurement from the user to the time when the operation input section 250 receives an input operation for finishing the measurement from the user. Further, the processing section 220 stores information pieces respectively indicating the calculated heart rate and the measured time period in the storage section 260 in response to a user's operation. Still further, the processing section 220 displays the information pieces indicating the heart rate and the time period on the display section 240 in response to a user's operation detected by the operation input section 250. The processing section 220 has an oscillator which generates a clock signal, and measures the time by using the clock signal.

The display section 240 has a display screen, and displays information output from the processing section 220, such as information indicating a hear rate or information indicating a time period, under the control of the processing section 220.

The operation input section 250 has a press button, and detects a user's operation of pressing the press button. The operation input section 250 outputs user's operation information indicating the detected user's operation to the processing section 220.

The storage section 260 stores a correspondence relationship between an AT speed and a time obtained when the user runs a predefined distance in the maximum exercise intensity, for each sex, for example. Here, the AT speed is a speed obtained when running is performed at an anaerobic threshold (AT) heart rate. The time obtained when the user runs in the maximum exercise intensity is a time, for example, in a case where the user runs a predefined distance at full speed. In the present embodiment, the time obtained when the user runs the predefined distance in the maximum exercise intensity is the user's best time of the user obtained when running 5 km as an example. In other words, in the present embodiment, as an example, the storage section 260 stores a correspondence relationship between an AT speed and the user's best time obtained when running 5 km, for each sex, for example. This correspondence relationship is determined on the basis of statistical data obtained from a plurality of subjects. In addition, the user's best time is not limited to the user's best time obtained when running 5 km, and may be the user's best time obtained when running other distances such as 10 km.

The inventor (hereinafter, referred to as the present inventor) of the present invention has found that a relationship between a running speed and a heart rate tends to be common to all subjects. More specifically, the present inventor has found that the variation in a heart rate relative to the variation in a running speed (that is, a slope in a case where the running speed is expressed by a transverse axis and the heart rate is expressed by a longitudinal axis) is not different depending on people but is approximately constant. From this founding, the variation in a heart rate relative to the variation relative to the variation in a running speed is a constant value which is common to all the subjects. Here, the variation in a heart rate relative to the variation in a running speed is, for example, a linear coefficient obtained when the heart rate is expressed by a linear function of the running speed. In addition, the variation in the heart rate relative to the variation in the running speed is a slope obtained when the running speed is expressed by a transverse axis, the heart rate is expressed by a longitudinal axis, and a relationship between the running speed and the heart rate is generated as a graph. In the present embodiment, as an example, the storage section 260 stores variation rate information indicating the variation in the heart rate relative to the variation in the running speed. Further, in the present embodiment, the storage section 260 stores the variation rate information indicating the variation in the heart rate relative to the variation in the running speed, but, without being limited thereto, the storage section 260 may store a correspondence relationship between predefined running speed and heart rate. For example, the storage section 260 may store a table in which the running speed is correlated with the heart rate.

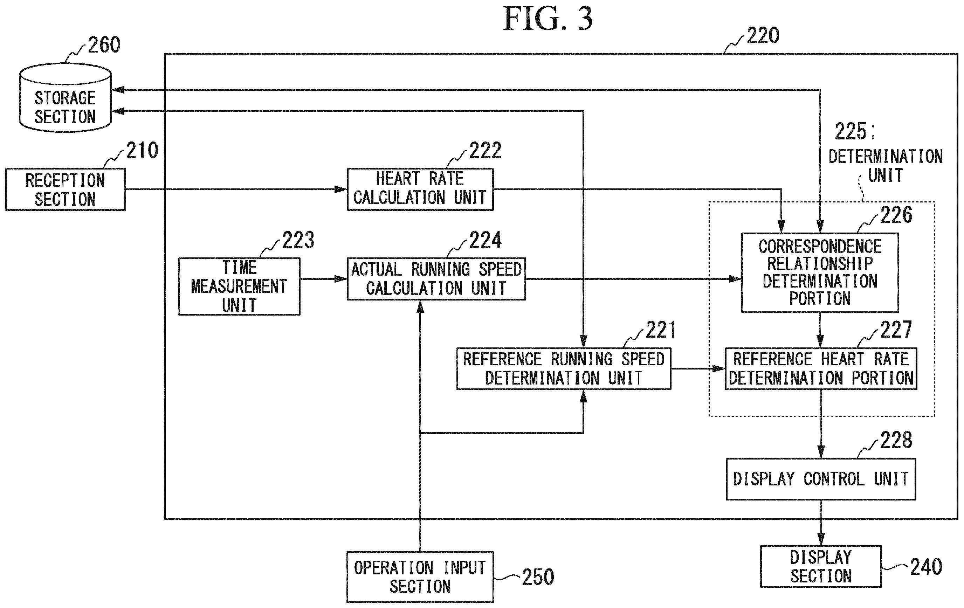

FIG. 3 is a schematic block diagram illustrating a functional configuration of the processing section 220 in the present embodiment. The processing section 220 includes a reference running speed determination unit 221, a heart rate calculation unit 222, a time measurement unit 223, an actual running speed calculation unit 224, a determination unit 225, and a display control unit 228.

The reference running speed determination unit 221 acquires, for example, the best time and a sex input by the user, from the user's operation information which is input via the operation input section 250. The reference running speed determination unit 221 refers to, for example, the correspondence relationship between an AT speed of the acquired sex and the best time at 5 km, stored in the storage section 260, and acquires an AT speed corresponding to the acquired best time. The reference running speed determination unit 221 outputs the acquired AT speed to a reference heart rate determination portion 227 (which will be described later) of the determination unit 225.

The heart rate calculation unit 222 calculates an actual running heart rate which is a heart rate obtained when the user actually runs, on the basis of heartbeat signals received by the reception section 210. Specifically, for example, in a case where the heartbeat signals are signals of 0 or 1, the heart rate calculation unit 222 calculates a heart rate as an actual running heart rate by counting signals indicating 1 for a minute among the received heartbeat signals. The heart rate calculation unit 222 outputs heart rate information indicating the calculated actual running heart rate, to a correspondence relationship determination portion 226 (which will be described later) of the determination unit 225.

The time measurement unit 223 measures the time. In the present embodiment, as an example, when the user runs 5 km while measuring heartbeats, the time measurement unit 223 measures the time required to fully run 5 km. The time measurement unit 223 outputs required time information indicating the measured time to the actual running speed calculation unit 224.

The actual running speed calculation unit 224 acquires a running distance input by the user, from user's operation information which is input via the operation input section 250. In the present embodiment, as an example, in a case where the user inputs "5 km" as a running distance before user runs 5 km while measuring heartbeats, the actual running speed calculation unit 224 acquires "5 km". The actual running speed calculation unit 224 calculates an actual running speed which is a speed during actual running, for example, by dividing the acquired running distance by the time indicated by the required time information which is input from the time measurement unit 223. Specifically, for example, in a case where the time required to fully run 5 km is 20 minutes, an actual running speed is 15 km/h obtained by dividing a result obtained by multiplying 5 indicated by "5 km" by 60, by 20. The actual running speed calculation unit 224 outputs actual running speed information indicating the calculated actual running speed to the correspondence relationship determination portion 226 (which will be described later) of the determination unit 225.

The determination unit 225 determines a correspondence relationship of a heart rate corresponding to a running speed, for example, on the basis of the actual running speed and the actual running heart rate. In addition, the determination unit 225 determines, for example, an AT heart rate corresponding to an AT speed on the basis of the determined correspondence relationship. Here, the determination unit 225 includes the correspondence relationship determination portion 226 and the reference heart rate determination portion 227.

The correspondence relationship determination portion 226 determines a correspondence relationship between a running speed and a heart rate on the basis of the running speed and the heart rate which have been actually measured for the user, and the predefined correspondence relationship between the variation in the running speed and the variation in the heart rate. Specifically, for example, the correspondence relationship determination portion 226 reads variation rate information stored in the storage section 260. Here, the variation rate information indicates a linear coefficient obtained when the heart rate is expressed by a linear function of the running speed. The correspondence relationship determination portion 226 calculates an intercept obtained when the heart rate is expressed by a linear function of the running speed, by using the actual running heart rate calculated by the heart rate calculation unit 222 and the actual running speed calculated by the actual running speed calculation unit 224. Consequently, a linear function of the running speed relative to the heart rate is uniquely determined. The correspondence relationship determination portion 226 outputs information indicating the linear function of the running speed relative to the heart rate to the reference heart rate determination portion 227.

The reference heart rate determination portion 227 determines an AT heart rate corresponding to an AT speed on the basis of the correspondence relationship determined by the correspondence relationship determination portion 226. Specifically, for example, the reference heart rate determination portion 227 calculates and determines the AT heart rate by assigning the AT speed acquired by the reference running speed determination unit to the linear function of the running speed relative to the heart rate. The reference heart rate determination portion 227 outputs information indicating the determined AT heart rate to the display control unit 228.

The display control unit 228 controls the display section 240 so that various information pieces are displayed on the display section 240. For example, the display control unit 228 determines a range (hereinafter, referred to as an AT zone) with the AT heart rate as a reference determined by the reference heart rate determination portion 227, and displays the generated AT zone. Specifically, for example, the display control unit 228 determines a range of the AT heart rate ± a predetermined value (for example, 10) as an example of the AT zone, and displays this range on the display section 240.

In addition, the display control unit 228 may determine a range of the AT heart rate ± the AT heart rate × a predetermined ratio (for example, 10%) as an example of the AT zone, and may display this range on the display section 240.

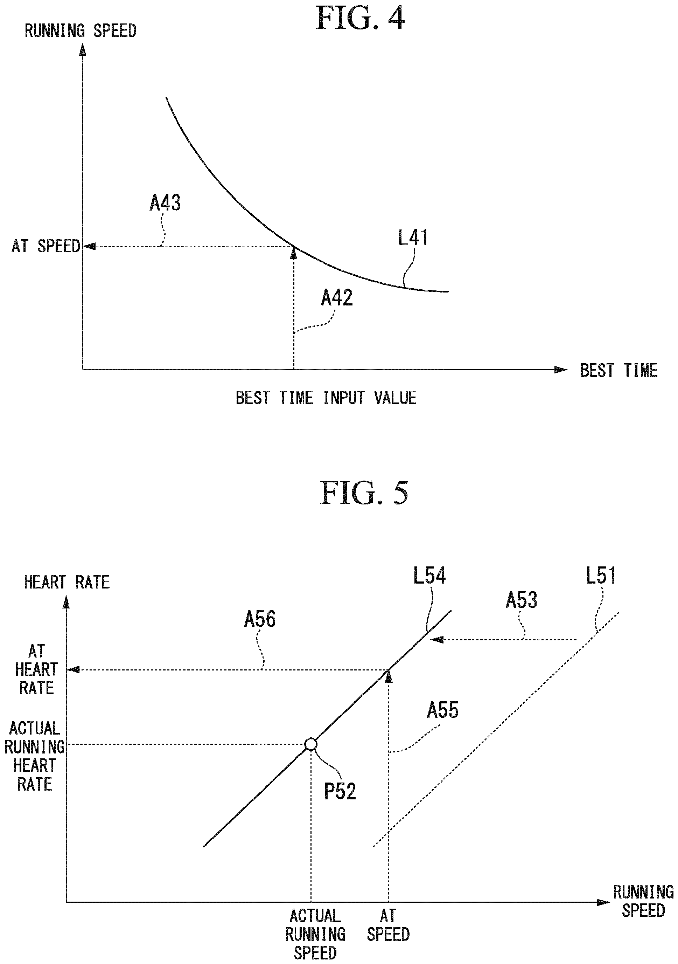

FIG. 4 is a diagram for explaining a concept of a process performed by the reference running speed determination unit 221. FIG. 4 illustrates an example of a curve L41 indicating a relationship between a running speed and the best time, stored in the storage section 260. Here, a longitudinal axis expresses a running speed, and a transverse axis expresses the best time. If the best time input by the user is given, a running speed corresponding to the given best time is uniquely determined in the curve L41 of the same figure as indicated by a dashed line arrow A42 and a dashed line arrow A43. The determined speed is the AT speed. Therefore, the reference running speed determination unit 221 can acquire a speed corresponding to a best time input value which is input by the user as the AT speed by referring to the relationship between the running speed and the best time.

FIG. 5 is a diagram for explaining a concept of a process performed by the determination unit 225. FIG. 5 illustrates a straight line L51 which has the variation in the heart rate relative to the variation in the running speed indicated by the variation rate information stored in the storage section 260, as a slope. Here, since only a value of the variation (that is, the slope of the straight line L51) in the heart rate relative to the variation in the running speed is a constant and is known, the straight line L51 is a straight line of which only the slope is determined and an intercept is any value. The straight line L51 is moved in parallel as indicated by a dashed line arrow A53 so as to pass through one point P52 which is determined by the actual running speed and the actual running heart rate, and thus a straight line L54 is obtained. Here, the actual running speed is obtained by dividing a distance which the user runs by the time required for the user to run the distance, and the actual running heart rate is, for example, an average heart rate obtained when the user runs the distance. This parallel movement process corresponds to a process of calculating an intercept of the straight line L54 in the correspondence relationship determination portion 226. The straight line L54 is an example of a correspondence relationship between a running speed and a heart rate, corrected by the correspondence relationship determination portion 226. As indicated by a dashed line arrow A55 and a dashed line arrow A56, if the AT speed calculated by the reference running speed determination unit 221 is given, an AT heart rate corresponding to the given AT speed is uniquely determined in the straight line L54. Therefore, the determination unit 225 calculates a linear function of the running speed relative to the heart rate, for example, on the basis of the predefined variation in the heart rate relative to the variation in the running speed, actual running speed, and actual running heart rate, and assigns the AT speed to the calculated linear function, so as to calculate the AT heart rate.

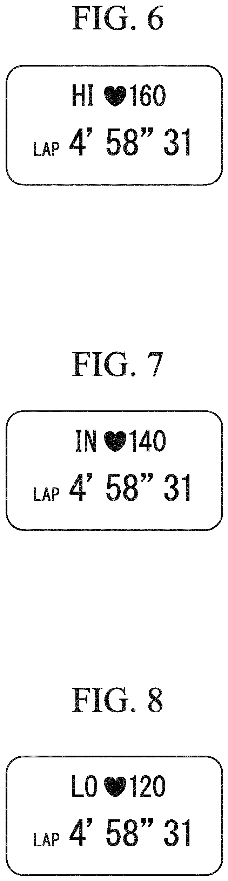

Next, a description will be made of examples of the display screen of the display section 240 with reference to examples of FIGS. 6 to 8. In common to the examples of FIGS. 6 to 8, information regarding the magnitude relation of the present heart rate relative to the AT zone, a heart mark, and the present heart rate are displayed on an upper end of the display screen. Here, in the examples of FIGS. 6 to 8, the AT zone is 120 to 140. In addition, the lap time is displayed as a chronograph measurement time on a lower end of the display screen. Further, display on the lower end is not limited to the lap time, and any information such as the split time, the present time, and a measurement time of a timer may be displayed.

FIG. 6 illustrates an example of the display screen in a case where the present heart rate exceeds the maximum heart rate of the AT zone. "HI" is displayed on the upper end of the display screen of FIG. 6 as the information regarding the magnitude relation of the present heart rate relative to the AT zone. Here, "HI" indicates that the present heart rate exceeds the maximum heart rate of the AT zone. In addition, "160" is displayed on the upper end of the display screen as the present heart rate.

FIG. 7 illustrates an example of the display screen in a case where the present heart rate is within the AT zone. "IN" is displayed on the upper end of the display screen of FIG. 7 as the information regarding the magnitude relation of the present heart rate relative to the AT zone. Here, "IN" indicates that the present heart rate is within the AT zone. In addition, "140" is displayed on the upper end of the display screen as the present heart rate.

FIG. 8 illustrates an example of the display screen in a case where the present heart rate is lower than the minimum AT zone. "LO" is displayed on the upper end of the display screen of FIG. 8 as the information regarding the magnitude relation of the present heart rate relative to the AT zone. Here, "LO" indicates that the present heart rate is lower than the minimum AT zone. In addition, "120" is displayed on the upper end of the display screen as the present heart rate.

To summarize the processes in the display control unit 228 in relation to FIGS. 6 to 8, the display control unit 228 determines a range (in the present embodiment, 120 to 140 as an example) of heartbeats with the AT heart rate as a reference, and displays the information (in the present embodiment, "HI", "IN", and "LO" as an example) regarding the magnitude relation of the heart rate relative to the determined range. The information regarding the magnitude relation of the heart rate relative to the determined range is information indicating, specifically, for example, whether the heart rate is within the range of the AT zone or is higher or lower than the range. Further, the display control unit 228 may display information (for example, the present heart rate such as 140) regarding a heart rate calculated by the heart rate calculation unit 222.

In addition, in the present embodiment, as an example, the display control unit 228 displays a set of the information (for example, "HI", "IN", and "LO") regarding the magnitude relation of the present heart rate relative to the AT zone, the heart mark, and the heart rate, but, without being limited thereto, at least the information regarding the magnitude relation of the present heart rate relative to the AT zone may be displayed. Further, "HI", "IN", and "LO" are only an example, and the present embodiment is not limited thereto, and any information may be displayed as long as the information indicates the magnitude relation of the present heart rate relative to the AT zone. Still further, the heart mark is only an example, and any mark may be used without being limited to this mark. Moreover, the display control unit 228 may change a type of mark, a shape of a mark, or a lighting state of a mark according to information regarding the magnitude relation of the present heart rate relative to the AT zone.

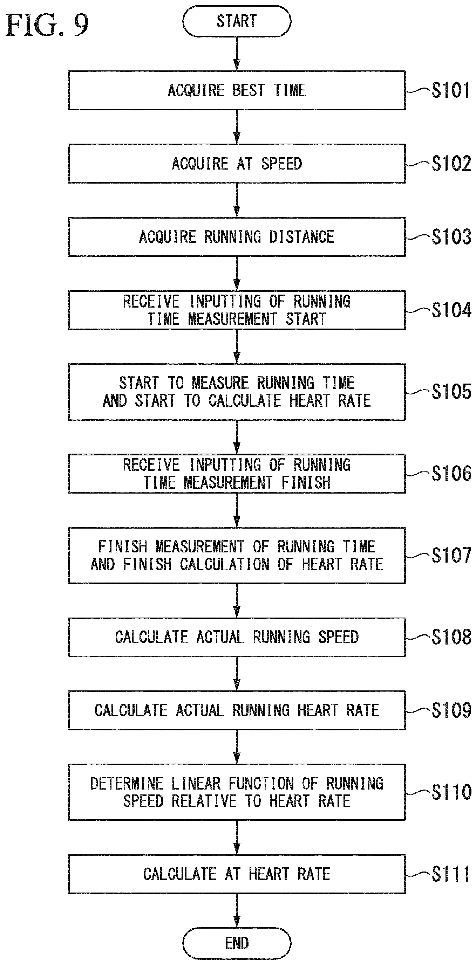

FIG. 9 is a flowchart illustrating an example of a process in the running watch system 1 according to the present embodiment.
(step S101) First, the reference running speed determination unit 221 acquires, for example, the user's best time at 5 km, on the basis of a user's operation received by the operation input section 250.
(step S102) Next, the reference running speed determination unit 221 acquires an AT speed corresponding to the acquired user's best time.
(step S103) Next, in a case where the user operates the operation input section 250 in order to input a predefined distance which the user will run before running, the operation input section 250 receives this user's operation. In addition, the actual running speed calculation unit 224 acquires the running distance on the basis of the user's operation received by the operation input section 250.
(step S104) Next, in a case where the user operates the operation input section 250 in order to give an instruction for starting a measurement of the running time simultaneously with the running, the operation input section 250 receives the inputting of the running time measurement start.
(step S105) Next, the time measurement unit 223 starts to measure the running time. In addition, the heart rate calculation unit 222 starts to calculate a heart rate on the basis of a heartbeat signal received by the reception section 210.
(step S106) Next, in a case where the user operates the operation input section 250 in order to give an instruction for finishing the measurement of the running time simultaneously with completion of the running of the predefined distance, the operation input section 250 receives the inputting of the running time measurement finish.
(step S107) The time measurement unit 223 finishes the measurement of the running time when the operation input section 250 receives the inputting of the running time measurement finish. In addition, along therewith, the heart rate calculation unit 222 finishes the calculation of a heart rate.
(Step S108) Next, the actual running speed calculation unit 224 calculates an actual running speed by dividing the distance input by the user by the running time.
(step S109) Next, the heart rate calculation unit 222 calculates an average heart rate which is an average of calculated heart rates as an actual running heart rate.
(step S110) Next, the correspondence relationship determination portion 226 reads the variation rate information from the storage section 260, and determines a linear function of the running speed relative to the heart rate by referring to the variation rate information, the actual running speed, and the actual running heart rate.
(step S111) Next, the reference heart rate determination portion 227 calculates an AT heart rate by assigning the AT speed to the determined linear function.

In addition, the operation input section 250 receives the operation of inputting a running distance before the user runs, but, without being limited thereto, the operation input section 250 may receive an operation of inputting the running distance after the user has completed the running. Further, the running watch 200 may additionally include a distance measurement unit, and the distance measurement unit may measure a running distance. Specifically, for example, a pace per pitch is input by the user and is stored in the storage section 260 in advance. In this case, the distance measurement unit may measures pitches, and may measure a running distance by multiplying the pace per pitch by the number of pitches.

As mentioned above, according to the present embodiment, a set of the actual running speed and the actual running heart rate is changed by a target user, and thus the determination unit 225 changes a linear function of the running speed relative to the heart rate depending on a target user. As a result, the determination unit 225 can change an AT heart rate depending on a target user. In addition, a set of an actual running speed and an actual running heart rate is changed when an actual running speed is measured even for the same target user, that is, depending on a state (for example, ability or condition) of the target user during the measurement. Therefore, even for the same target user, a linear function of the running speed relative to the heart rate is changed depending on a state of the user when an actual running speed and an actual running heart rate are measured. As a result, the determination unit 225 can determine an AT heart rate depending on a state of the target user during the measurement.

In addition, as an example of the related art, an AT heart rate is determined according to age, and thus an accurate AT heart rate cannot be acquired in a case of a person with a low heart rate or a high heart rate. In contrast, according to the present embodiment, the determination unit 225 determines a linear function by using an actual running speed obtained through one running measurement for a target user, an actual running heart rate during the running measurement, and the variation in the heart rate relative to the variation in the running speed based on the statistical data (refer to FIG. 5). Consequently, a linear function can be obtained for each target user, and thus an AT heart rate is changed depending on the linear function. For this reason, the determination unit 225 calculates an AT heart rate according to a set of the actual running speed of the target user and the actual running heart rate during the running measurement and can thus more accurately determine an AT heart rate even for a person with a low heart rate or a high heart rate.

In addition, the reference running speed determination unit 221 acquires an AT speed corresponding to the best time of a target user by referring to a relationship between the running speed and the best time (refer to FIG. 4). For this reason, since the best time differs depending on a target user, the reference running speed determination unit 221 can acquire a different AT speed for each target user.

Further, since the best time is changed due to improvement in the ability associated with training or the like or a reduction in the ability caused by a decrease in an amount of training even for the same target user, an AT speed changes according to the change in the ability even for the same target user. Therefore, the reference running speed determination unit 221 can acquire an AT speed in proportion to the present ability of a target user. As mentioned above, since the determination unit 225 determines an AT heart rate corresponding to the AT speed by referring to the determined linear function, if the reference running speed determination unit 221 changes the AT speed depending on a change in the present ability of the target user, the determination unit 225 changes the AT heart rate according thereto. Therefore, the processing section 220 can determine an AT heart rate corresponding to the present ability of the target user.

In addition, in the present embodiment, it has been found by the present inventor that there is little individual difference in the variation in the heart rate relative to the variation in the running speed, and thus a single variation in the heart rate relative to the variation in the running speed is predefined and is common to all users. In addition, the determination unit 225 determines an equation for expressing a heart rate as a linear function of a running speed, by using an actual running speed obtained through one running measurement for a certain distance in a user (hereinafter, referred to as a target user) as a measurement target and an actual running heart rate during the running measurement. The determination unit 225 determines the linear function as mentioned above and can thus determine an AT heart rate corresponding to an AT speed of the target user by using the linear function. Consequently, since one running measurement is enough, it is possible to reduce time and effort of a user, and a burden of the user, caused by acquisition of an AT heart rate.

In addition, in the present embodiment, the determination unit 225 determines an AT heart rate on the basis of an AT speed, but, without being limited thereto, the determination unit 225 may determine the maximum heart rate on the basis of an AT speed, and may determine a cardiorespiratory ability enhancement heart rate which is a heart rate set to enhance the cardiorespiratory ability. Here, the cardiorespiratory ability enhancement heart rate is a heart rate set to enhance the cardiorespiratory ability, and differs depending on a user's ability. A user exercises at the cardiorespiratory ability enhancement heart rate and can thus increase the cardiorespiratory ability. Further, the cardiorespiratory ability enhancement heart rate is a heart rate between the maximum heart rate and an AT heart rate.

Still further, the determination unit 225 may determine one of an AT heart rate, a cardiorespiratory ability enhancement heart rate, and the maximum heart rate on the basis of not an AT speed but a cardiorespiratory ability enhancement speed which is a speed set to enhance the cardiorespiratory ability. Furthermore, the determination unit 225 may determine one of an AT heart rate, a cardiorespiratory ability enhancement heart rate, and the maximum heart rate on the basis of not an AT speed but the maximum speed which is a speed obtained when running in the maximum exercise intensity.

In addition, in the present embodiment, the reference running speed determination unit 221 acquires an AT speed corresponding to the best time of a user at 5 km on the basis of a correspondence relationship between the best time at 5 km and an AT speed, and the present invention is not limited thereto. Not only the best time at 5 km but also the best time at other distances such as 10 km may be used. Further, not only the time required to fully run a predefined distance in the maximum exercise intensity, such as the best time, but also the time required to fully run the distance at predetermined exercise intensity may be used. Still further, not only an AT speed but also a cardiorespiratory ability enhancement speed or the maximum speed may be used. Therefore, a correspondence relationship may be a correspondence relationship between the time required for a user to fully run a predefined distance at predetermined exercise intensity and a reference running speed. In this case, the reference running speed determination unit 221 may determine a reference running speed corresponding to the time required for the user to fully run the predefined distance at the predetermined exercise intensity on the basis of the correspondence relationship.

In addition, in the present embodiment, the reference running speed determination unit 221 uses the correspondence relationship between the time required to fully run a predefined distance in a predetermined exercise intensity and a reference running speed, and the present invention is not limited thereto. The reference running speed determination unit 221 may use a correspondence relationship between a speed during running at predetermined exercise intensity and a reference running speed. In this case, the reference running speed determination unit 221 may determine a reference running speed corresponding to a speed obtained when a user runs at the predetermined exercise intensity. Further, the reference running speed determination unit 221 may use a correspondence relationship between a distance obtained when running is performed for a predetermined time period at predetermined exercise intensity and a reference running speed. In this case, the reference running speed determination unit 221 may determine a reference running speed corresponding to a distance obtained when a user runs a predetermined time period at the predetermined exercise intensity. To summarize the above description, the reference running speed determination unit 221 may determine a reference running speed corresponding to a predetermined exercise ability index value of a user on the basis of a correspondence relationship between the predetermined exercise ability index value and the reference running speed. Here, the predetermined exercise ability index value is any one of the time required to fully run a predefined distance at predetermined exercise intensity, a speed obtained when running is performed at predetermined exercise intensity, and a distance obtained when running is performed for a predetermined time period at predetermined exercise intensity.

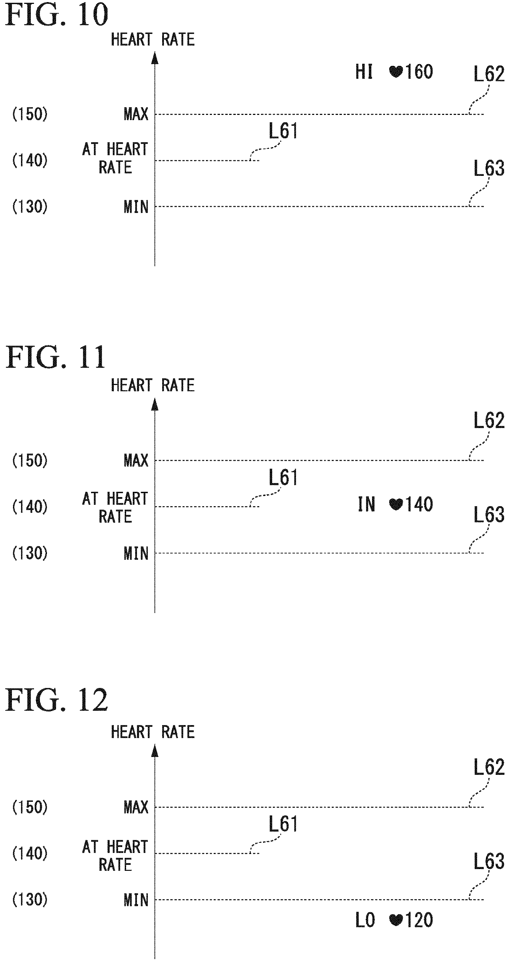

Next, application examples of the display screen of the display section 240 will be described by using examples of FIGS. 10 to 12. FIG. 10 illustrates a first application example of the display screen of the display section 240. In common to the application examples of FIGS. 10 to 12, a longitudinal axis expresses a heart rate. In common to the examples of FIGS. 10 to 12, a heart rate "140" is displayed as an AT heart rate on the display screen, and a dashed line L61 indicating the heart rate "140" is displayed. In addition, in common to the examples of FIGS. 10 to 12, 150 which is obtained by adding 10 to the AT heart rate on the display screen is displayed as the maximum value MAX of a heart rate of a zone (hereinafter, referred to as an AT zone) with the AT heart rate as a reference, and a dashed line L62 indicating the heart rate "150" is displayed. Further, in common to examples of FIGS. 10 to 12, 130 which is obtained by subtracting 10 from the AT heart rate on the display screen is displayed as the minimum value MIN of a heart rate of the AT zone, and a dashed line L63 indicating the heart rate "130" is displayed.

In FIG. 10, "HI", a heart mark, and "160" which is the present heart rate are displayed at a position in the longitudinal axis direction, corresponding to the present heart rate "160" on the display screen. Here, "HI" indicates that a heart rate exceeds the maximum value MAX of a heart rate of the AT zone. As mentioned above, the display control unit 228 displays the AT zone with a heart rate expressed by the longitudinal axis, and displays the heart mark and the like over the AT zone in a case where the present heart rate exceeds the maximum value MAX of a heart rate of the AT zone. Consequently, since a user can identify that the present heart rate exceeds the AT zone, the user can easily recognize that the present heart rate exceeds the AT zone. Further, since "HI" is displayed, the user can easily recognize that the present heart rate exceeds the AT zone.

FIG. 11 illustrates a second application example of the display screen of the display section. In FIG. 11, "IN", a heart mark, and "140" which is the present heart rate are displayed at a position in the longitudinal axis direction, corresponding to the present heart rate "140" on the display screen. Here, "IN" indicates that a heart rate is within the AT zone. As mentioned above, the display control unit 228 displays the AT zone with a heart rate expressed by the longitudinal axis, and displays the heart mark and the like within the AT zone in a case where the present heart rate is within the AT zone. Consequently, the user can easily recognize that the present heart rate is within the AT zone. Further, since "IN" is displayed, the user can easily recognize that the present heart rate is within the AT zone.

FIG. 12 is a third application example of the display screen of the display section. In FIG. 12, "LO", a heart mark, and "120" which is the present heart rate are displayed at a position in the longitudinal axis direction, corresponding to the present heart rate "120" on the display screen. Here, "LO" indicates that a heart rate is lower than the minimum value MIN of a heart rate of the AT zone. As mentioned above, the display control unit 228 displays the AT zone with a heart rate expressed by the longitudinal axis, and displays the heart mark and the like under the AT zone in a case where the present heart rate is lower than the minimum value MIN of a heart rate of the AT zone. Consequently, the user can easily recognize that the present heart rate is lower than the AT zone. Further, since "LO" is displayed, the user can easily recognize that the present heart rate is lower than the AT zone.

In addition, in FIGS. 10 to 12, the display control unit 228 may change a display position of the heart mark or the like depending on a height of the present heart rate. For example, the display control unit 228 may display the heart mark and the like at a position in the longitudinal axis direction, corresponding to the present heart rate. Consequently, a user can easily recognize how far a value of the present heart rate is away from the AT zone, or at which position a value of the present heart rate is located in the AT zone. Further, in FIGS. 10 to 12, a heart rate is expressed by the longitudinal axis, but a heart rate may be expressed by the transverse axis. In addition, in the present application example, a heart rate and the like are displayed at a position of the longitudinal axis corresponding to the present heart rate, but the present invention is not limited thereto. For example, in a case where the present heart rate exceeds the maximum value MAX of a heart rate of the AT zone, the display control unit 228 may display the heart rate and the like at any position (for example, any position in FIG. 10 further upward located than the dashed line L62 of FIG. 10) above the displayed AT zone. In addition, for example, in a case where the present heart rate is within the AT zone, the display control unit 228 may display the heart rate and the like at any position (for example, any position between the dashed line L63 and the dashed line L62 of FIG. 11) in the AT zone. In addition, for example, in a case where the present heart rate is lower than the AT zone, the display control unit 228 may display the heart rate and the like at any position (for example, any position below the dashed line L63 of FIG. 12) below the AT zone.

### <Modification Example 1>

Next, Modification Example 1 of the present embodiment will be described. In Modification Example 1, a correspondence relationship between the best time and a cardiorespiratory ability enhancement running speed is determined, and the storage section 260 stores the correspondence relationship between the cardiorespiratory ability enhancement running speed and the best time. The cardiorespiratory ability enhancement running speed is a running speed which is determined in order to enhance the cardiorespiratory ability, and is a speed between an AT speed and the maximum running speed. The maximum running speed is a running speed obtained when a user runs in the maximum exercise intensity. In this case, the reference running speed determination unit 221 may determine a cardiorespiratory ability enhancement running speed corresponding to the best time on the basis of the correspondence relationship between the cardiorespiratory ability enhancement running speed and the best time, stored in the storage section 260. In this case, the determination unit 225 may determine one of an AT heart rate, a cardiorespiratory ability enhancement heart rate, and the maximum heart rate on the basis of the cardiorespiratory ability enhancement running speed.

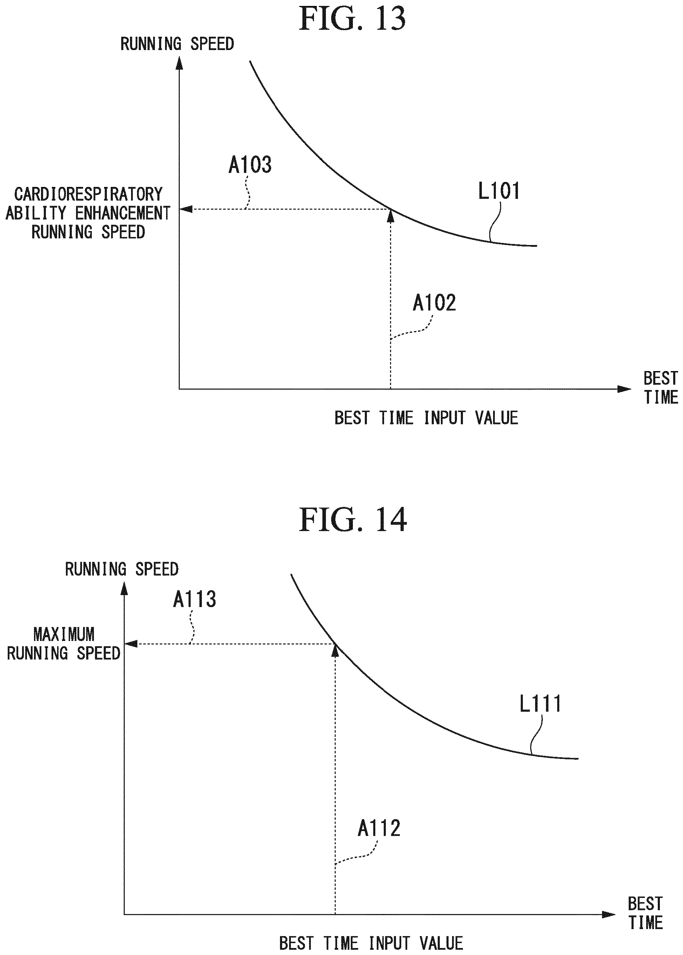

FIG. 13 is a diagram for explaining a concept of a process performed by the reference running speed determination unit 221 in Modification Example 1. FIG. 13 illustrates an example of a curve L101 indicating a relationship between a cardiorespiratory ability enhancement running speed and the best time, stored in the storage section 260. Here, a longitudinal axis expresses a running speed, and a transverse axis expresses the best time. If a best time input value which is input by the user is given, a running speed corresponding to the given best time is uniquely determined in the curve L101 of the same figure as indicated by a dashed line arrow A102 and a dashed line arrow A103. The determined running speed is the cardiorespiratory ability enhancement running speed. Therefore, the reference running speed determination unit 221 can acquire a speed corresponding to a best time input value which is input by the user as the cardiorespiratory ability enhancement running speed by referring to the relationship between the cardiorespiratory ability enhancement running speed and the best time.

### <Modification Example 2>

Next, Modification Example 2 of the present embodiment will be described. In Modification Example 2, a correspondence relationship between the best time and the maximum running speed is determined, and the storage section 260 stores the correspondence relationship between the maximum running speed and the best time. In this case, the reference running speed determination unit 221 may determine the maximum running speed corresponding to the best time on the basis of the correspondence relationship between the maximum running speed and the best time, stored in the storage section 260. In this case, the determination unit 225 may determine one of an AT heart rate, a cardiorespiratory ability enhancement heart rate, and the maximum heart rate on the basis of the maximum running speed.

FIG. 14 is a diagram for explaining a concept of a process performed by the reference running speed determination unit 221 in Modification Example 2. FIG. 14 illustrates an example of a curve L111 indicating a relationship between the maximum running speed and the best time, stored in the storage section 260. Here, a longitudinal axis expresses a running speed, and a transverse axis expresses the best time. If the best time input by the user is given, a running speed corresponding to the given best time is uniquely determined in the curve L111 of the same figure as indicated by a dashed line arrow A112 and a dashed line arrow A113. The determined running speed is the maximum running speed. Therefore, the reference running speed determination unit 221 can acquire a speed corresponding to a best time input value which is input by the user as the maximum running speed by referring to the relationship between the maximum running speed and the best time.

As described above, to summarize of the processes of the running watch 200 in the present embodiment, and Modification Examples 1 and 2, the determination unit 225 may determine a reference heart rate (for example, an AT heart rate, a cardiorespiratory ability enhancement heart rate, or the maximum heart rate) which is used as a reference of a heart rate on the basis of a reference running speed (for example, an AT speed, a cardiorespiratory ability enhancement speed, or the maximum speed) which is used as a reference of the exercise ability of a user, and a predefined correspondence relationship between the variation in a running speed and the variation in a heart rate. Here, the correspondence relationship is a relationship in which the variation in the heart rate relative to the variation in the running speed is constant. Since the variation in the heart rate relative to the variation in the running speed is constant regardless of a user, a reference heart rate can be determined on the basis of the reference running speed and the correspondence relationship between the running speed and the heart rate.
Consequently, if the reference running speed and the correspondence relationship between the running speed and the heart rate are known, a reference heart rate can be determined, and thus it is possible to reduce a user's burden when determining the reference heart rate.

More specifically, the reference running speed determination unit 221 determines a reference running speed corresponding to the time required for a user to fully run a predefined distance at predetermined exercise intensity on the basis of a correspondence relationship between the time required to fully run the predefined distance at the predetermined exercise intensity and the reference running speed. Consequently, the reference running speed determination unit 221 can determine a reference running speed corresponding to a user's ability. The determination unit 225 determines a correspondence relationship between the running speed and the heart rate on the basis of a set of a running speed and a heart rate, actually measured for a user, and a correspondence relationship between the variation in the running speed relative to the variation in the heart rate, and determines a reference heart rate corresponding to a reference running speed on the basis of the determined correspondence relationship. Consequently, the determination unit 225 can determine the correspondence relationship between the running speed and the heart rate depending on a state (for example, ability or condition) of the user and can thus determine a reference heart rate according to the state of the user. In addition, if a running speed and a heart rate are measured once, a reference heart rate can be determined, and thus it is possible to reduce time and effort, and a burden of the user.

The heart rate calculation unit 222 calculates a heart rate on the basis of a heartbeat signal regarding heartbeats of a user.

The display control unit 228 determines a range of heartbeats with an AT heart rate as a reference, displays the generated range on the display section, and displays information regarding the heart rate calculated by the heart rate calculation unit at a position corresponding to a height of the heart rate. Consequently, the user can identify whether or not the present heart rate enters the range of the AT heart rate, and thus the user can easily realize running (for example, a marathon race or training) for keeping the AT heart rate.

In addition, in the present embodiment, a correspondence relationship between an AT speed and the time obtained when a user runs a predefined distance in the maximum exercise intensity is stored for each sex, but, a single correspondence relationship between an AT speed and the time obtained when a user runs a predefined distance in the maximum exercise intensity may be stored regardless of a sex. A correspondence relationship in this case may be an average of a male correspondence relationship and a female correspondence relationship.

In addition, a sex is input as physical information of a user, and height and weight may also be input. Further, a relationship between an AT speed and the best time is stored in storage section 260 for each height or weight, and the processing section 220 may determine an AT speed by referring to the relationship. Still further, not only height but also a length of a partial body (for example, a length of the leg) may be input. In this case, a relationship between an AT speed and the best time is stored in storage section 260 for each length of a partial body, and the processing section 220 may determine an AT speed by referring to the relationship.

In addition, a program for executing the respective processes in the processing section 220 of the present embodiment may be recorded on a computer readable recording medium, and the program recorded on the recording medium may be read to a computer system and be executed, so that the above-described various processes related to the processing section 220 may be performed.

Further, the "computer system" described here may be one including an OS or hardware such as a peripheral device. Furthermore, the "computer system" is assumed to also include home page providing circumstances (or display circumstances) if the WWW system is used. Moreover, the "computer readable recording medium" refers to a flexible disk, a magneto-optical disc, a ROM, a writable nonvolatile memory such as a flash memory, a portable medium such as a CD-ROM, or a storage device such as a hard disk built in the computer system.

In addition, the "computer readable recording medium" also includes one which holds a program for a specific time such as a volatile memory (for example, dynamic random access memory (DRAM)) of the computer system, which becomes a server or a client when the program is transmitted via a network such as the Internet or a communication line such as a telephone line. Further, the program may be transmitted from a computer system in which the program is stored in a storage device or the like to other computer systems via a transmission medium, or using a transmission wave in the transmission medium. Here, the "transmission medium" which transmits the program refers to a medium having a function of transmitting information, including a network (communication network) such as the Internet or a communication line such as a telephone line. Furthermore, the program may be used to realize some of the above-described functions. Moreover, the program may be a so-called differential file (differential program) which can realize the above-described functions in combination with a program which has already been recorded in the computer system.

### REFERENCE SYMBOL LIST

- 1: RUNNING WATCH SYSTEM
- 100: CHEST STRAP
- 110: SENSOR SECTION
- 120: TRANSMISSION SECTION
- 200: RUNNING WATCH
- 210: RECEPTION SECTION
- 220: PROCESSING SECTION
- 221: REFERENCE RUNNING SPEED DETERMINATION UNIT
- 222: HEART RATE CALCULATION UNIT
- 223: TIME MEASUREMENT UNIT
- 224: ACTUAL RUNNING SPEED CALCULATION UNIT
- 225: DETERMINATION UNIT
- 226: CORRESPONDENCE RELATIONSHIP DETERMINATION PORTION
- 227: REFERENCE HEART RATE DETERMINATION PORTION
- 228: DISPLAY CONTROL UNIT
- 240: DISPLAY SECTION
- 250: OPERATION INPUT SECTION
- 260: STORAGE SECTION

## Claims

1. An electronic apparatus comprising:
a determination unit (225) that determines a reference heart rate used as a reference of a heart rate on the basis of a reference running speed used as a reference of exercise ability of a user, wherein the determination unit
determines a correspondence relationship (L54) between running speed and heart rate from variation rate information of variation in heart rate relative to variation in running speed, the variation rate information being a slope (L51) that is constant regardless of the user, to intercept a point (P52) determined by an actual running heart rate and an actual running speed measured for the user, and
determines the reference heart rate from the reference running speed using the correspondence relationship between running speed and heart rate.

2. The electronic apparatus according to claim 1, further comprising:
a reference running speed determination unit that determines the reference running speed on the basis of a predetermined correspondence relationship between exercise ability index value and reference running speed,
wherein the determination unit determines a reference heart rate on the basis of the reference running speed determined by the reference running speed determination unit.

3. The electronic apparatus according to claim 2, wherein the exercise ability index value is any one of a time required to fully run a predefined distance at predetermined exercise intensity, a speed obtained when running is performed at predetermined exercise intensity, and a distance obtained when running is performed for a predetermined time period at predetermined exercise intensity.

4. The electronic apparatus according to claim 3, whereat the predetermined exercise intensity is maximum exercise intensity.

5. The electronic apparatus according to any one of claims 1 to 4, wherein the reference running speed is an AT speed, a cardiorespiratory ability enhancement speed which is a speed set to enhance cardiorespiratory ability, or the maximum speed which is a speed obtained when running is performed in maximum exercise intensity.

6. The electronic apparatus according to any one of claims 1 to 5, wherein the reference heart rate is an AT heart rate, a cardiorespiratory ability enhancement heart rate which is a heart rate set to enhance cardiorespiratory ability, or maximum heart rate.

7. The electronic apparatus according to claim 6, wherein the cardiorespiratory ability enhancement heart rate is between the AT heart rate and the maximum heart rate.

8. The electronic apparatus according to claim 6 or 7, further comprising:
a heart rate calculation unit (222) that calculates a heart rate on the basis of a heartbeat signal regarding heartbeats of the user; and
a display control unit (228) that determines a range of heartbeats with the AT heart rate as a reference, and displays at least information regarding a magnitude relation of the heart rate relative to the determined range.

9. A non-transitory computer-readable recording medium storing a program comprising instructions for causing a computer to execute a method comprising:
a step of determining a reference heart rate used as a reference of a heart rate on the basis of a reference running speed used as a reference of exercise ability of a user, the step comprising
determining a correspondence relationship (L54) between running speed and heart rate from variation rate information of variation in heart rate relative to variation in running speed, the variation rate information being a slope (L51) that is constant regardless of the user, to intercept with a point (P52) determined by an actual running heart rate and an actual running speed measured for the user, and
determining the reference heart rate from the reference running speed using the correspondence relationship between running speed and heart rate.

## Patentansprüche

1. Elektronische Vorrichtung, umfassend:
eine Ermittlungseinheit (225), die eine Referenzherzfrequenz, die als eine Referenz einer Herzfrequenz verwendet wird, auf der Basis einer Referenzlaufgeschwindigkeit ermittelt, die als eine Referenz einer Trainingsfähigkeit eines Benutzers verwendet wird, wobei die Ermittlungseinheit
ein Entsprechungsverhältnis (L54) zwischen Laufgeschwindigkeit und Herzfrequenz aus Variationsrateninformationen einer Variation in Herzfrequenz relativ zu einer Variation in Laufgeschwindigkeit ermittelt, wobei die Variationsrateninformationen eine Neigung (L51) sind, die unabhängig von einem Benutzer konstant ist, die einen Punkt (P52) schneiden soll, der aus einer tatsächlichen Laufherzfrequenz und einer tatsächlichen Laufgeschwindigkeit, die für den Benutzer gemessen werden, ermittelt wird, und
die Referenzherzfrequenz aus der Referenzlaufgeschwindigkeit unter Verwendung des Entsprechungsverhältnisses zwischen Laufgeschwindigkeit und Herzfrequenz ermittelt.

2. Elektronische Vorrichtung nach Anspruch 1, weiter umfassend:
eine Referenzlaufgeschwindigkeit-Ermittlungseinheit, die die Referenzlaufgeschwindigkeit auf der Basis eines vorgegebenen Entsprechungsverhältnisses zwischen Trainingsfähigkeit-Indexwert und Referenzlaufgeschwindigkeit ermittelt,
wobei die Ermittlungseinheit eine Referenzherzfrequenz auf der Basis der Referenzlaufgeschwindigkeit ermittelt, die durch die Referenzlaufgeschwindigkeit-Ermittlungseinheit ermittelt wird.

3. Elektronische Vorrichtung nach Anspruch 2, wobei der Trainingsfähigkeit-Indexwert eine von einer Zeit, die erforderlich ist, um eine vordefinierte Strecke bei vorgegebener Trainingsintensität vollständig zu laufen, einer Geschwindigkeit, die erhalten wird, wenn der Lauf bei einer vorgegebenen Trainingsintensität durchgeführt wird, und einer Strecke, die erhalten wird, wenn der Lauf für einen vorgegebenen Zeitraum bei vorgegebener Trainingsintensität durchgeführt wird, ist.

4. Elektronische Vorrichtung nach Anspruch 3, wobei die vorgegebene Trainingsintensität eine maximale Trainingsintensität ist.

5. Elektronische Vorrichtung nach einem der Ansprüche 1 bis 4, wobei die Referenzlaufgeschwindigkeit eine AT-Geschwindigkeit, eine Geschwindigkeit zur Verbesserung der kardiorespiratorischen Fähigkeit, die eine Geschwindigkeit ist, die zum Verbessern der kardiorespiratorischen Fähigkeit eingestellt wird, oder eine maximale Geschwindigkeit ist, die eine Geschwindigkeit ist, die erhalten wird, wenn der Lauf bei maximaler Trainingsintensität durchgeführt wird.

6. Elektronische Vorrichtung nach einem der Ansprüche 1 bis 5, wobei die Referenzherzfrequenz eine AT-Herzfrequenz, eine Herzfrequenz zur Verbesserung der kardiorespiratorischen Fähigkeit, die eine Herzfrequenz ist, die zum Verbessern der kardiorespiratorischen Fähigkeit eingestellt wird, oder eine maximale Herzfrequenz ist.

7. Elektronische Vorrichtung nach Anspruch 6, wobei die Herzfrequenz zur Verbesserung der kardiorespiratorischen Fähigkeit zwischen der AT-Herzfrequenz und der maximalen Herzfrequenz liegt.

8. Elektronische Vorrichtung nach Anspruch 6 oder 7, weiter umfassend:
eine Herzfrequenzberechnungseinheit (222), die eine Herzfrequenz auf der Basis eines Herzschlagsignals bezüglich Herzschlägen des Benutzers berechnet; und
eine Anzeigesteuerungseinheit (228), die einen Bereich von Herzschlägen mit der AT-Herzfrequenz als Referenz ermittelt und mindestens Informationen bezüglich eines Größenverhältnisses der Herzfrequenz relativ zu dem ermittelten Bereich anzeigt.

9. Nicht transitorisches, computerlesbares Aufzeichnungsmedium, das ein Programm speichert, das Anweisungen umfasst, die einen Computer veranlassen, ein Verfahren auszuführen, umfassend:
einen Schritt zum Ermitteln einer Referenzherzfrequenz, die als eine Referenz einer Herzfrequenz verwendet wird, auf der Basis einer Referenzlaufgeschwindigkeit, die als eine Referenz einer Trainingsfähigkeit eines Benutzers verwendet wird, wobei der Schritt umfasst
Ermitteln eines Entsprechungsverhältnisses (L54) zwischen Laufgeschwindigkeit und Herzfrequenz aus Variationsrateninformationen einer Variation in Herzfrequenz relativ zu einer Variation in Laufgeschwindigkeit, wobei die Variationsrateninformationen eine Neigung (L51) sind, die unabhängig von einem Benutzer konstant ist, die einen Punkt (P52) schneiden soll, der aus einer tatsächlichen Laufherzfrequenz und einer tatsächlichen Laufgeschwindigkeit, die für den Benutzer gemessen werden, ermittelt wird, und
Ermitteln der Referenzherzfrequenz aus der Referenzlaufgeschwindigkeit unter Verwendung des Entsprechungsverhältnisses zwischen Laufgeschwindigkeit und Herzfrequenz.

## Revendications

1. Dispositif électronique comprenant :
une unité de détermination (225) déterminant une fréquence cardiaque de référence utilisée comme référence d'une fréquence cardiaque sur la base d'une vitesse de course de référence utilisée comme référence de capacité d'exercice d'un utilisateur, dans lequel l'unité de détermination
détermine une relation de correspondance (L54) entre une vitesse de course et une fréquence cardiaque à partir d'informations de taux de variation concernant une variation dans la fréquence cardiaque par rapport à une variation dans la vitesse de course, les informations de taux de variation consistant en une pente (L51), laquelle est constante quel que soit l'utilisateur, pour intercepter un point (P52) déterminé par une fréquence cardiaque de course réelle et une vitesse de course réelle mesurées pour l'utilisateur, et
détermine la fréquence cardiaque de référence à partir de la vitesse de course de référence à l'aide de la relation de correspondance entre la vitesse de course et la fréquence cardiaque.

2. Dispositif électronique selon la revendication 1, comprenant en outre :
une unité de détermination de vitesse de course de référence, laquelle détermine la vitesse de course de référence sur la base d'une relation de correspondance prédéterminée entre une valeur d'indice de capacité d'exercice et une vitesse de course de référence,
dans lequel l'unité de détermination détermine une fréquence cardiaque de référence sur la base de la vitesse de course de référence déterminée par l'unité de détermination de vitesse de course de référence.

3. Dispositif électronique selon la revendication 2, dans lequel la valeur d'indice de capacité d'exercice est l'une quelconque parmi un temps nécessaire pour parcourir entièrement une distance prédéfinie à une intensité d'exercice prédéterminée, une vitesse obtenue lorsque la course est effectuée à une intensité d'exercice prédéterminée, et une distance obtenue lorsque la course est effectuée pour une durée prédéterminée à une intensité d'exercice prédéterminée.

4. Dispositif électronique selon la revendication 3, dans lequel l'intensité d'exercice prédéterminée est une intensité d'exercice maximale.

5. Dispositif électronique selon l'une quelconque des revendications 1 à 4, dans lequel la vitesse de course de référence est une vitesse AT, une vitesse d'amélioration de la capacité cardiorespiratoire, laquelle est une vitesse fixée pour améliorer une capacité cardiorespiratoire, ou la vitesse maximale, laquelle est une vitesse obtenue lorsque la course est effectuée à une intensité d'exercice maximale.

6. Dispositif électronique selon l'une quelconque des revendications 1 à 5, dans lequel la fréquence cardiaque de référence est une fréquence cardiaque AT, une fréquence cardiaque d'amélioration de la capacité cardiorespiratoire, laquelle est une fréquence cardiaque fixée pour améliorer une capacité cardiorespiratoire, ou une fréquence cardiaque maximale.

7. Dispositif électronique selon la revendication 6, dans lequel la fréquence cardiaque d'amélioration de la capacité cardiorespiratoire se situe entre la fréquence cardiaque AT et la fréquence cardiaque maximale.

8. Dispositif électronique selon la revendication 6 ou 7, comprenant en outre :
une unité de calcul de fréquence cardiaque (222) calculant une fréquence cardiaque sur la base d'un signal de battements cardiaques relatif aux battements cardiaques de l'utilisateur ; et
une unité de commande d'affichage (228) déterminant une plage de battements cardiaques avec la fréquence cardiaque AT comme référence, et affichant au moins des informations concernant un rapport de grandeur de la fréquence cardiaque par rapport à la plage déterminée.

9. Support d'enregistrement non transitoire lisible par ordinateur, stockant un programme comprenant des instructions destinées à amener un ordinateur à mettre en œuvre un procédé comprenant :
une étape de détermination d'une fréquence cardiaque de référence utilisée comme référence d'une fréquence cardiaque sur la base d'une vitesse de course de référence utilisée comme référence de la capacité d'exercice d'un utilisateur, l'étape comprenant :
la détermination d'une relation de correspondance (L54) entre une vitesse de course et une fréquence cardiaque à partir d'informations de taux de variation concernant une variation dans la fréquence cardiaque par rapport à une variation dans la vitesse de course, les informations de taux de variation consistant en une pente (L51), laquelle est constante quel que soit l'utilisateur, pour intercepter un point (P52) déterminé par une fréquence cardiaque de course réelle et une vitesse de course réelle mesurées pour l'utilisateur, et
la détermination de la fréquence cardiaque de référence à partir de la vitesse de course de référence à l'aide de la relation de correspondance entre la vitesse de course et la fréquence cardiaque.
